# EUROPEAN PATENT SPECIFICATION

(11) **EP 3 782 057 B1**
(45) Date of publication and mention of the grant of the patent: **25.06.2025**
(21) Application number: 19788649.2
(22) Date of filing: 17.04.2019
(51) Int. Cl.: G16B 25/20, G16B 30/20, G16B 35/10, G06F 3/0486, G06F 3/04845, G06F 3/04883, C12N 15/63

(54) **DNA CONSTRUCT DESIGN SYSTEM**
DESIGNSYSTEM FÜR DNA-KONSTRUKT
SYSTÈME DE CONCEPTION DE CONSTRUCTION D'ADN

(30) Priority: 17.04.2018 WO PCT/CN2018/083359
(43) Date of publication of application: 24.02.2021
(73) Proprietor: Nanjing GenScript Biotech Co., Ltd., Nanjing, Jiangsu 211100 (CN)
(72) Inventor: WANG, Ke, Piscataway, New Jersey 08854 (US); ZHANG, Lihua, Nanjing, Jiangsu 211100 (CN); SHENG, Xia, Nanjing, Jiangsu 211100 (CN); QIN, Yu, Piscataway, New Jersey 08854 (US); LI, Shiyue, Nanjing, Jiangsu 211100 (CN); SHEN, Qiunan, Nanjing, Jiangsu 211100 (CN); DUAN, Junwei, Nanjing, Jiangsu 211100 (CN); XU, Hongchao, Nanjing, Jiangsu 211100 (CN)
(74) Representative: Schmidt, Christian
(86) International application number: PCT/CN2019/083042
(87) International publication number: WO 2019/201276

(56) References cited:
- CN-A- 103 907 117
- US-A- 5 965 444
- US-A1- 2007 043 516
- VILLALOBOS A ET AL: "Gene Designer: a synthetic biology tool for constructing artificial DNA segments", BMC BIOINFORMATICS, BIOMED CENTRAL , LONDON, GB, vol. 7, no. 1, 6 June 2006 (2006-06-06), pages 1 - 8, XP021013796, ISSN: 1471-2105, DOI: 10.1186/1471-2105-7-285
- LOPAREV A ET AL: "BacPack", TANGIBLE, EMBEDDED, AND EMBODIED INTERACTION, ACM, 2 PENN PLAZA, SUITE 701 NEW YORK NY 10121-0701 USA, 20 March 2017 (2017-03-20), pages 111 - 120, XP058326587, ISBN: 978-1-4503-4676-4, DOI: 10.1145/3024969.3025000
- "Plant Synthetic Promoters : Methods and Protocols", vol. 1482, 25 August 2016, SPRINGER NEW YORK, ISBN: 978-1-4939-6396-6, ISSN: 1064-3745, article COLL A ET AL: "GenoCAD Plant Grammar to Design Plant Expression Vectors for Promoter Analysis: Methods and Protocols", pages: 219 - 232, XP093231584, DOI: 10.1007/978-1-4939-6396-6_14

## Description

### FIELD OF THE INVENTION

The present disclosure relates generally to a computer system, and more specifically to techniques for providing a DNA construct design system.

### BACKGROUND OF THE INVENTION

A DNA construct is an artificially constructed segment of nucleic acid created by inserting target DNA fragment(s) into a vector backbone, and is a vehicle for carrying target DNA fragment(s) into a target tissue or cell. Artificial plasmids are commonly used vectors for amplifying the target DNA fragment(s) in host organisms. Upon propagation in the host organisms, plasmids may then be isolated using various methods of plasmid preparation. Plasmids are widely used as vectors in biological studies involving gene function analysis, protein expression and genome editing.

The process of DNA construct design involves selecting a right position to insert a target DNA fragment. Due to the complexity of DNA construct design, it is desirable to conduct the design process using computer software. However, many existing computer software for designing DNA construct provide a user experience that is inflexible, unintuitive, and/or unguided. For example, the functionalities provided by the computer software are often limited, restricting construct design to only templates that has been known to work while providing no flexibility to design construct without pre-designed template. As another example, users, especially those who do not have advanced knowledge in DNA construct design or in the particular software, often find the user interface confusing and cumbersome to operate. Further still, these computer software lack adequate artificial intelligence to guide a user in the design process, for example, by automatically detecting errors in the user's design and providing tailored suggestions.

Villalobos et al. (2006), "Gene Designer: a synthetic biology tool for constructing artificial DNA segments", BMC BIOINFORMATICS, 7(1):1-8 discloses the software "Gene Designer" for fast and easy design of synthetic DNA segments. Likewise, WO2007022260A2 discloses software tools for designing and manipulating sequence elements in order to design polynucleotides encoding custom genetic constructs. However, neither of this prior art discloses convenient manipulation via drag-and-drop.

### BRIEF SUMMARY OF THE INVENTION

The invention is defined in the appended claims.

The present disclosure relates to a computer-implemented DNA construct design system providing a flexible, intuitive, and guided user experience. In some aspects, the system enables "part-based" construct design which, as discussed below, maximizes flexibility by freeing the user from having to design DNA constructs within a predefined framework or template. Further, the system provides an intuitive and natural user interface, for example, by allowing the user to conduct the design using simple inputs such as drag and drop (e.g., using a mouse or a touch-enable display screen). Further, the system automatically detects errors in the user's design via built-in design checking algorithms and provides notifications and suggestions accordingly.

In some embodiments, there is provided a computer-implemented method of designing a DNA construct that comprises: at an electronic device with a display, receiving an input selecting a vector backbone, wherein the vector backbone comprises a plurality of functional parts; in response to receiving the input selecting the vector backbone, displaying a graphical representation of the vector backbone; receiving an input selecting one or more functional parts of the plurality of functional parts of the vector backbone; after receiving the input selecting one or more functional parts on the vector backbone, receiving a drag-and-drop input comprising an indication of a functional part; in response to receiving the drag-and-drop input, updating the vector backbone based on the functional part indicated in the drag-and-drop input and the selected one or more functional parts of the plurality of functional parts of the vector backbone; and displaying a graphical representation of the updated vector backbone.

In some embodiments, the drag-and-drop input comprises a click and drag input made with a mouse.

In some embodiments, the drag-and-drop input comprises a tap and drag input made with a finger on a touch-sensitive display.

According to the invention, the graphical representation of the selected vector backbone includes a plasmid map, or a sequence map, or a combination thereof.

In one alternative according to the invention, the one or more functional parts includes an existing gene on the vector backbone, and updating the vector backbone comprises replacing the existing gene with the functional part indicated in the drag-and-drop input.

In the other alternative according to the invention, the one or more functional parts includes one or more cloning sites on the vector backbone, and updating the vector backbone comprises inserting the functional part indicated in the drag-and-drop input at a cloning site of the one or more cloning sites on the vector backbone.

In some embodiments, the electronic device receives an input including a search term corresponding to a functional part; and identifies one or more search results based on a plurality of databases.

In some embodiments, the plurality of databases includes a user-specific database, a system-specific database, a public database, or any combination thereof.

In some embodiments, while displaying the graphical representation of the updated vector backbone, the electronic device receives an input indicative of an error-checking request; in response to receiving the input indicative of the error-checking request, the electronic device identifies an error with the updated vector backbone; and provides an output indicative of the identified error.

In some embodiments, in response to receiving the drag-and-drop input, automatically the electronic device identifies the functional part indicated in the drag-and-drop input based on a plurality of databases; and based on the identifying, displays a graphical representation of the functional part indicated in the drag-and-drop input in accordance with one or more visual characteristics associated with the functional part.

In some embodiments, the plurality of functional parts include: a promoter; a gene of interest; a terminator; a tag; an antibiotic resistance; a cloning site; an origin; a reporter gene; a coding sequence ("CDS"); an activator; an enhancer; an intron; an repressor; a signal sequence; a terminal repeat sequence; a linker; or any combination thereof.

In some embodiments, there is provided an electronic device that comprises a display; one or more processors; a memory; and one or more programs. The one or more programs are stored in the memory and configured to be executed by the one or more processors, the one or more programs including instructions for: receiving an input selecting a vector backbone, wherein the vector backbone comprises a plurality of functional parts; in response to receiving the input selecting the vector backbone, displaying a graphical representation of the vector backbone; receiving an input selecting one or more functional parts of the plurality of functional parts of the vector backbone; after receiving the input selecting one or more functional parts on the vector backbone, receiving a drag-and-drop input comprising an indication of a functional part; in response to receiving the drag-and-drop input, updating the vector backbone based on the functional part indicated in the drag-and-drop input and the selected one or more functional parts of the plurality of functional parts of the vector backbone; and displaying a graphical representation of the updated vector backbone.

In further aspects also described herein, there is provided a non-transitory computer-readable storage medium that stores one or more programs, the one or more programs comprising instructions, which when executed by one or more processors of an electronic device having a display, cause the electronic device to: receive an input selecting a vector backbone, wherein the vector backbone comprises a plurality of functional parts; in response to receiving the input selecting the vector backbone, display a graphical representation of the vector backbone; receive an input selecting one or more functional parts of the plurality of functional parts of the vector backbone; after receiving the input selecting one or more functional parts on the vector backbone, receive a drag-and-drop input comprising an indication of a functional part; in response to receiving the drag-and-drop input, update the vector backbone based on the functional part indicated in the drag-and-drop input and the selected one or more functional parts of the plurality of functional parts of the vector backbone; and display a graphical representation of the updated vector backbone.

In some embodiments, there is provided a computer-implemented method of error-checking a user-edited DNA construct that comprises: receiving, at an electronic device, an error-checking request on the user-edited DNA construct; in response to receiving the input, identifying a set of sequences in the user-edited DNA construct, wherein the set of sequences is not present in the original DNA construct; identifying a presence of one or more coding sequences (e.g., ORF and/or putative CDS) by comparing the set of sequences with a plurality of databases; identifying one or more errors in the identified one or more coding sequences based on a plurality of predefined rules; and displaying an output indicative of the one or more errors, wherein the user-edited DNA construct is edited based on an original DNA construct according to some of the above methods.

In some embodiments, the output comprises: a textual output; a graphical output; an auditory output; or any combination thereof.

In some embodiments, identifying one or more errors comprises identifies one or more invalid characters in the set of sequences.

In some embodiments, the plurality of databases includes a user-specific database, a system-specific database, a public database, or any combination thereof.

In some embodiments, identifying the set of sequences in the user-edited DNA construct comprises: identifying a first sequence not present in the original DNA construct; identifying a second sequence not present in the original DNA construct, wherein the second sequence is within a predetermined distance from the first sequence in the user-edited DNA construct; merging the first sequence and the second sequence to obtain a third sequence, wherein the third sequence is part of the set of sequences.

In some embodiments, identifying one or more errors comprises: determining whether a stop codon is present within a coding sequence of the one or more coding sequences.

In some embodiments, identifying one or more errors comprises: determining whether a start codon is present before a coding sequence of the one or more coding sequences.

In some embodiments, identifying one or more errors comprises: determining whether two coding sequences of the one or more coding sequences are within a predefined distance in the user-edited DNA construct and whether the two coding sequences are of a same direction.

In some embodiments, in accordance with a determination that two coding sequences of the one or more coding sequences are within a predefined distance in the user-edited DNA construct and that the two coding sequences are of a same direction: the electronic device determines whether the two coding sequences are in-frame.

In some embodiments, in accordance with a determination that two coding sequences of the one or more coding sequences are within a predefined distance in the user-edited DNA construct and that the two coding sequences are of a same direction: the electronic device determines whether a stop codon is present between the two coding sequences.

In some embodiments, identifying one or more errors comprises: determining whether a promoter is present within a predefined distance with a coding sequence of the one or more coding sequences.

In some embodiments, in accordance with a determination that a promoter is present within a predefined distance with a coding sequence of the one or more coding sequences: the electronic device determines whether the promoter is of a same direction as the coding sequence.

In some embodiments, there is provided an electronic device that comprises one or more processors; a memory; and one or more programs. The one or more programs are stored in the memory and configured to be executed by the one or more processors, the one or more programs including instructions for: receiving an error-checking request on a user-edited DNA construct; in response to receiving the input, identifying a set of sequences in the user-edited DNA construct, wherein the set of sequences is not present in the original DNA construct; identifying a presence of one or more coding sequences by comparing the set of sequences with a plurality of databases; identifying one or more errors in the identified one or more coding sequences based on a plurality of predefined rules; and displaying an output indicative of the one or more errors, wherein the user-edited DNA construct is edited based on an original DNA construct according to some of the above methods.

In further aspects also described herein, there is provided a non-transitory computer-readable storage medium stores one or more programs, the one or more programs comprising instructions, which when executed by one or more processors of an electronic device having a display, cause the electronic device to: receive an error-checking request on a user-edited DNA construct, wherein the user-edited DNA construct is edited based on an original DNA construct; in response to receiving the input, identify a set of sequences in the user-edited DNA construct, wherein the set of sequences is not present in the original DNA construct; identify a presence of one or more coding sequences by comparing the set of sequences with a plurality of databases; identify one or more errors in the identified one or more coding sequences based on a plurality of predefined rules; and display an output indicative of the one or more errors.

### BRIEF DESCRIPTION OF THE DRAWINGS

For a better understanding of the various described embodiments, reference should be made to the Description of Embodiments below, in conjunction with the following drawings in which like reference numerals refer to corresponding parts throughout the figures.
FIG. 1A depicts an exemplary user interface of an electronic device in accordance with some embodiments;
FIG. 1B depicts an exemplary user interface of an electronic device in accordance with some embodiments;
FIG. 1C depicts an exemplary user interface of an electronic device in accordance with some embodiments;
FIG. 1D depicts an exemplary user interface of an electronic device in accordance with some embodiments;
FIG. 1E depicts an exemplary user interface of an electronic device in accordance with some embodiments;
FIG. 1F depicts an exemplary user interface of an electronic device in accordance with some embodiments;
FIG. 1G depicts an exemplary user interface of an electronic device in accordance with some embodiments;
FIG. 1H depicts an exemplary user interface of an electronic device in accordance with some embodiments;
FIG. 1I depicts an exemplary user interface of an electronic device in accordance with some embodiments;
FIG. 1J depicts an exemplary user interface of an electronic device in accordance with some embodiments;
FIG. 1K depicts an exemplary user interface of an electronic device in accordance with some embodiments;
FIG. 1L depicts an exemplary user interface of an electronic device in accordance with some embodiments;
FIG. 2A depicts an exemplary user interface of an electronic device in accordance with some embodiments;
FIG. 2B depicts an exemplary user interface of an electronic device in accordance with some embodiments;
FIG. 2C depicts an exemplary user interface of an electronic device in accordance with some embodiments;
FIG. 3 illustrates an exemplary database structure in accordance with some embodiments;
FIG. 4A illustrates an overview of an exemplary error-checking process in accordance with some embodiments;
FIG. 4B illustrates steps in an exemplary error-checking process in accordance with some embodiments;
FIG. 4C illustrates steps in exemplary error-checking process in accordance with some embodiments;
FIG. **4D** illustrates steps in an exemplary error-checking process in accordance with some embodiments;
FIG. 5A illustrates an exemplary process for providing a DNA construct design system in accordance with some embodiments;
FIG. 5B depicts an exemplary process for providing a DNA construct design system in accordance with some embodiments;
FIG. 6 depicts an exemplary electronic device in accordance with some embodiments.

### DETAILED DESCRIPTION OF THE INVENTION

The following description sets forth exemplary methods, parameters, and the like. It should be recognized, however, that such description is not intended as a limitation on the scope of the present disclosure but is instead provided as a description of exemplary embodiments.

Although the following description uses terms "first," "second," etc. to describe various elements, these elements should not be limited by the terms. These terms are only used to distinguish one element from another. For example, a first graphical representation could be termed a second graphical representation, and, similarly, a second graphical representation could be termed a first graphical representation, without departing from the scope of the various described embodiments. The first graphical representation and the second graphical representation are both graphical representations, but they are not the same graphical representation.

The terminology used in the description of the various described embodiments herein is for the purpose of describing particular embodiments only and is not intended to be limiting. As used in the description of the various described embodiments and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

The term "if" is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

The present disclosure relates to a computer-implemented DNA construct design system providing a flexible, intuitive, and guided user experience. In some embodiments, the system enables "part-based" construct design which, as discussed below, maximizes flexibility by freeing the user from having to design DNA constructs within a predefined framework or template. Further, the system provides an intuitive and natural user interface, for example, by allowing the user to conduct the design using simple inputs such as drag and drop (e.g., using a mouse or a touch-enable display screen). Further, the system automatically detects errors in the user's design via built-in design checking algorithms and provides notifications and suggestions accordingly.

For purposes of the present disclosure, the basic unit of a DNA construct is called as a "part" or a "functional element". Parts are functionally ordered on the construct to realize functions like prorogation in bacteria. Examples of a part include, but are not limited to: a promoter, a gene of interest, a terminator, a tag, an antibiotic resistance, a cloning site, an origin, a reporter gene, a coding sequence ("CDS"), an activator, an enhancer, an intron, an repressor, a signal sequence , terminal repeat sequence, a linker, or any combination or subcombination thereof. Parts can be categorized by their functions. The exemplary categories are listed in Table 1.

**Table 1 - Main Categories of Parts**

| Category | Description | Examples |
|---|---|---|
| Promoter | Initiate transcription | CMV promoter, T7 promoter |
| Gene of interest | ORFs | TP53 |
| Terminator | Stop transcription | bGH polyA |
| Tag | Protein tags are peptide sequences attached to proteins. Tags are often removable by chemicals or enzymatic digestion. | GST tag, His tag, Flag tag |
| Antibiotic resistance | Gene confers an antibiotic trait for artificial selection | Ampicillin resistance gene, Hygromycin resistance gene |
| Multiple cloning site | A cluster of restriction enzyme sites | / |
| Origin | Help construct replicate independently in host cell | pBR322 origin, pUC origin |
| Reporter gene | Gene confers a trait that can be easily identified or detected for artificial selection | GFP, mCherry, GUS |

Construct can replicate in the host independently triggered by origin component. The cassette of bacteria selection marker, consisting of promoter and CDS of antibiotic gene, facilitates positive colony selection, by expressing an antibiotic gene (i.e., Ampicillin, Kanamycin). Multiple cloning site (MCS) is designed for cloning purpose, including inserting a target DNA fragment by restriction enzyme digestion. Regarding protein expression purpose, promoter (i.e., T7 promoter in E.coli expression vector, CMV in Mammalian expression vector) and terminator is harbored on the backbone at the upstream and downstream of coding sequence to initiate and stop transcription, respectively. In between is the target DNA segment inserted which is often coding sequence (CDS) such as ORF (Open Reading Frame) which is stated as a continuous stretch of amino acid codons, typically starting with a start codon (ATG) and ending with a stop codon (TAA, TAG, or TGA). To detect or isolate target proteins, tags (His tag, Flag tag) or reporters (i.e., GFP) are usually fused with target proteins, N-terminus or C-terminus. In other words, target protein is fused with a tag sequence or a reporter gene to express a fusion protein. To ensure the expression and function of the fusion protein, every single nucleotide should be in order to promise the correct translation, which is termed as "in-frame". In contrast, frame shift caused by in-del (insertion or deletion) will not lead to the target fusion protein as expected.

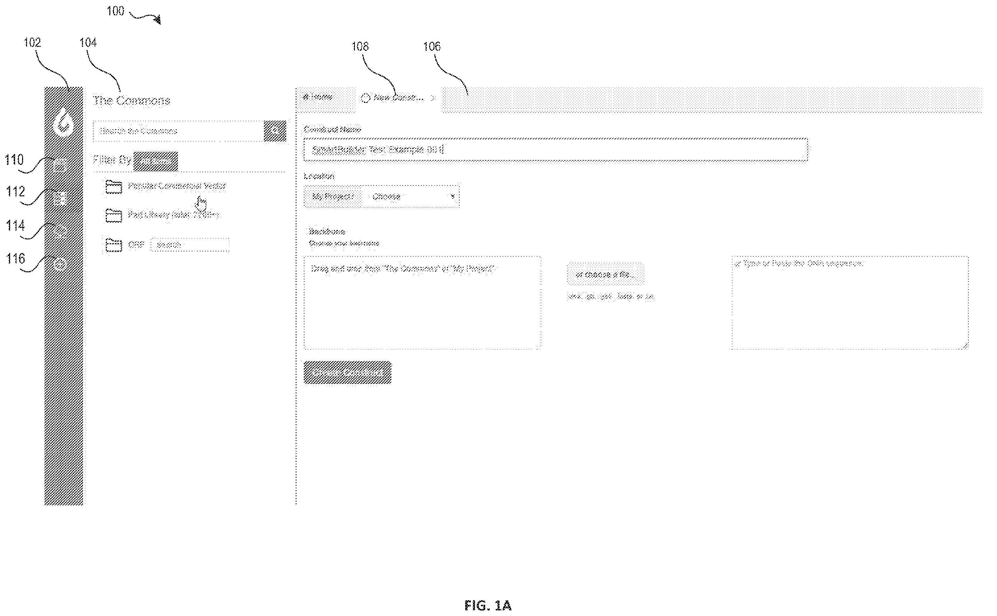

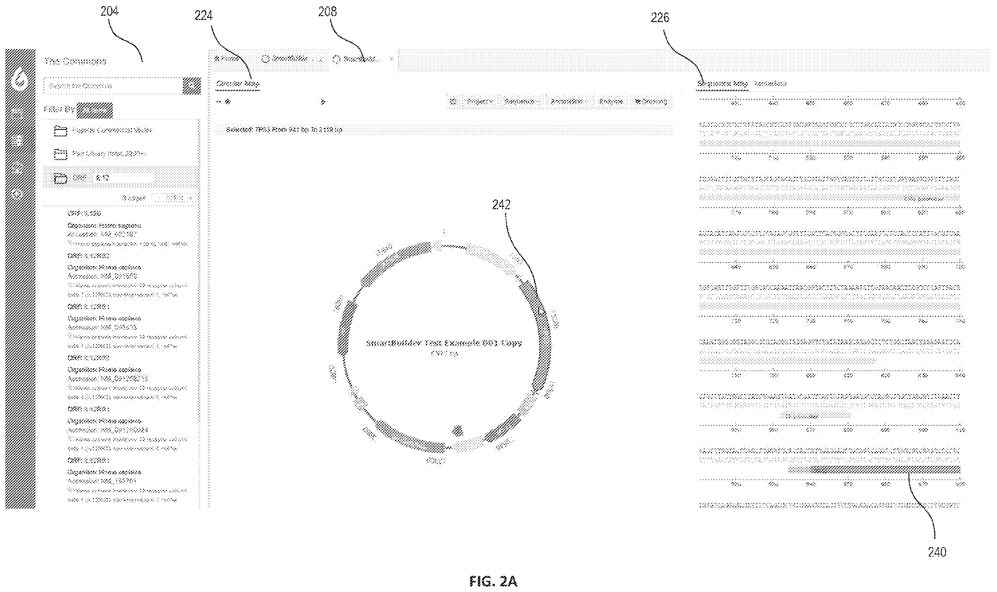

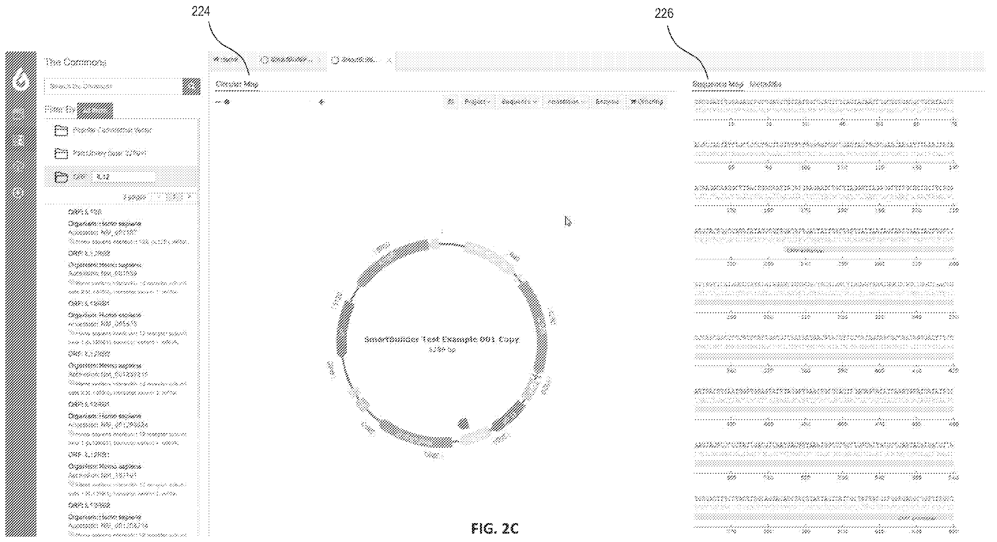

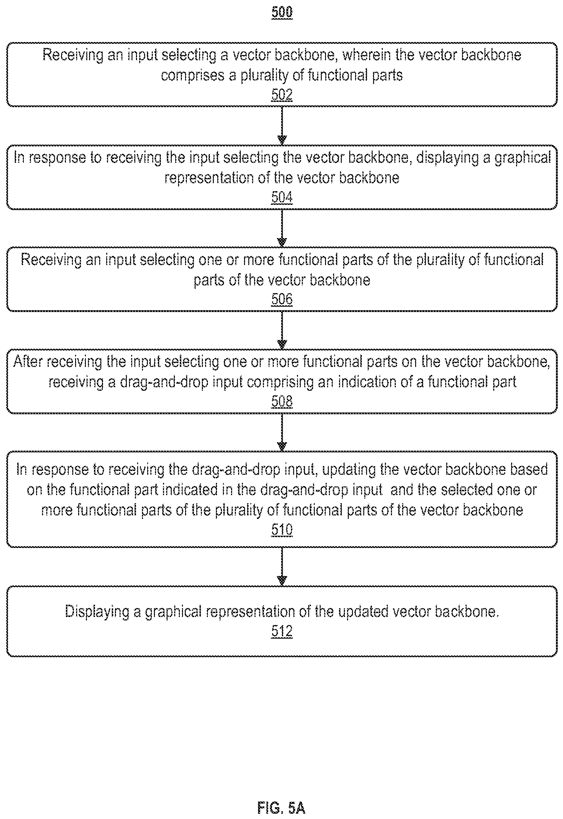

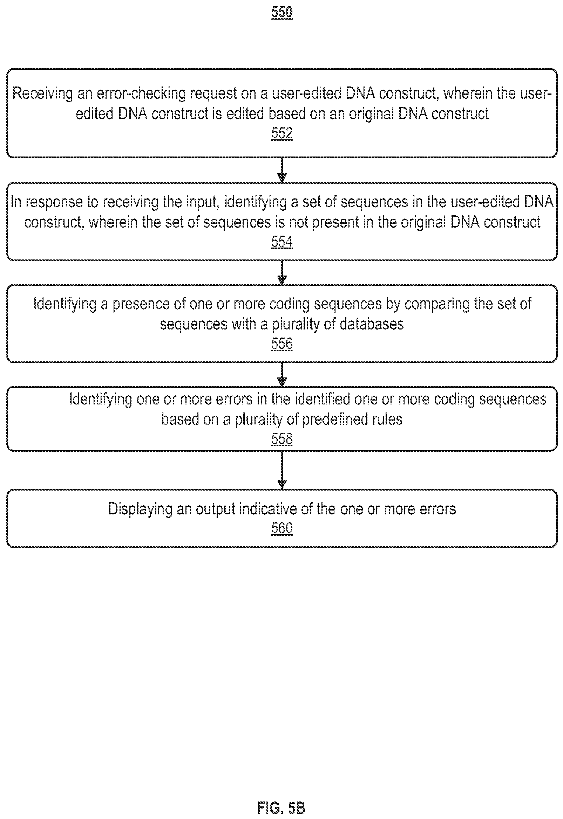

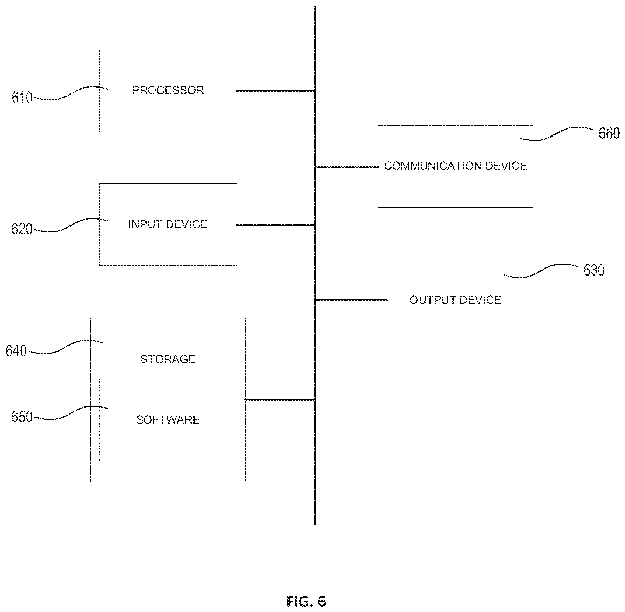

FIGS. 1A-1K and 2A-C illustrate exemplary techniques including exemplary user interfaces ("UI") for providing a DNA construct design system in accordance with some embodiments. These figures are also used to illustrate the processes described below, including the exemplary processes in FIGS. 5A-B. The exemplary techniques and processes can be implemented using a variety of electronic devices with displays (e.g., touchscreen displays), such as laptops, desktops, tablets, portable/wearable devices, or a combination thereof. An exemplary computing device is illustrated in FIG. 6.

FIG. 1A depicts an exemplary user interface 100 of a DNA construct design system. The user interface 100 includes a side menu section 102, a database navigation section 104, and a working space section 106. The side menu section 102 includes multiple icons for accessing various functionalities and databases of the system. In particular, icons 110, 112, and 114 respectively correspond to three groups of databases, "My Projects", "The Commons", and "Global Resources", which are described in further detail below. Icon 115 corresponds to an "Add New" feature, which allows the user to start a new construct design or a new insert design.

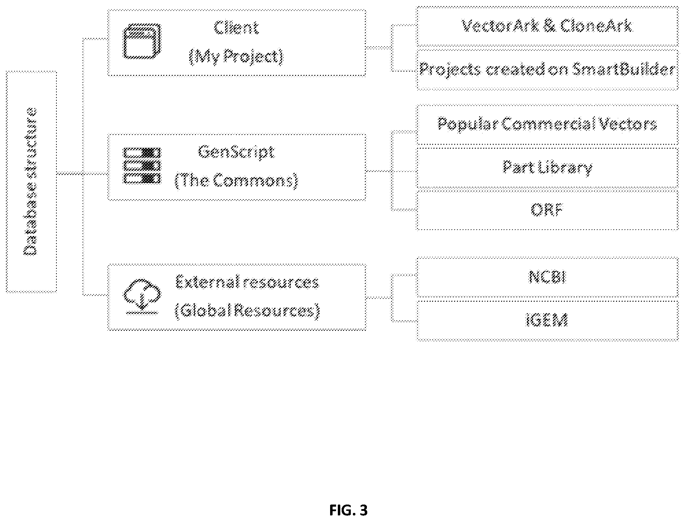

The DNA construct design system provides a variety of databases to maximize the flexibility, simplicity, and efficiency of the design process. FIG. 3 illustrates an exemplary database structure of the system. As depicted, the database structure includes three groups of databases: user-specific databases (referred to as "My Projects"), system-specific databases (referred to as "The Commons"), and public databases (referred to as "Global Resource").

The user-specific databases include previous projects associated with (e.g., previously saved to) the user's account, including archived vectors and clones (referred to as "VectorArk" and "CloneArk" respectively), thus allowing the user to design and manufacture constructs based on previous orders. The system-specific databases are further grouped into three categories: "Popular Commercial Vector", "Part Library", and "ORF". "Popular Commercial Vector" includes commonly used vectors, listed and grouped by host (e.g., Bacterium, Baculovirus, Mammalian, Pichia and S. cerevisiae). Most can be employed to protein expression, while cloning vectors (pUC series) are also in the list. "Part Library" includes a unique collection of functional parts, such as frequently-used or validated vector elements and parts published in peer reviewed papers. "ORF" provides quick access to search for ORF. In some examples, the system-specific databases are periodically maintained and updated by the operators of the system. The public databases include a variety of external databases that the system makes available to the user. In the depicted example, the public databases include NCBI, which is a database center for biomedical and genomics research, and iGEM, which provides a collection of validated parts used in International Genetically Engineered Machine (iGEM) Competition.

Turning back to FIG. 1A, in the depicted example, the user selects the icon 112 (corresponding to "The Commons") in the side menu section 102. Accordingly, the database navigation section 104 displays a nested list of system-specific databases for the user to view, search, and select from. Further, the working space section 106 displays a tab user interface 108 for creating a new DNA construct. The tab user interface 108 prompts the user to specify a name for the new construct and an initial vector backbone for the new construct. In some examples, the tab user interface 108 can be launched by selecting the icon 116 (corresponding to "Add New") in the side menu section 102.

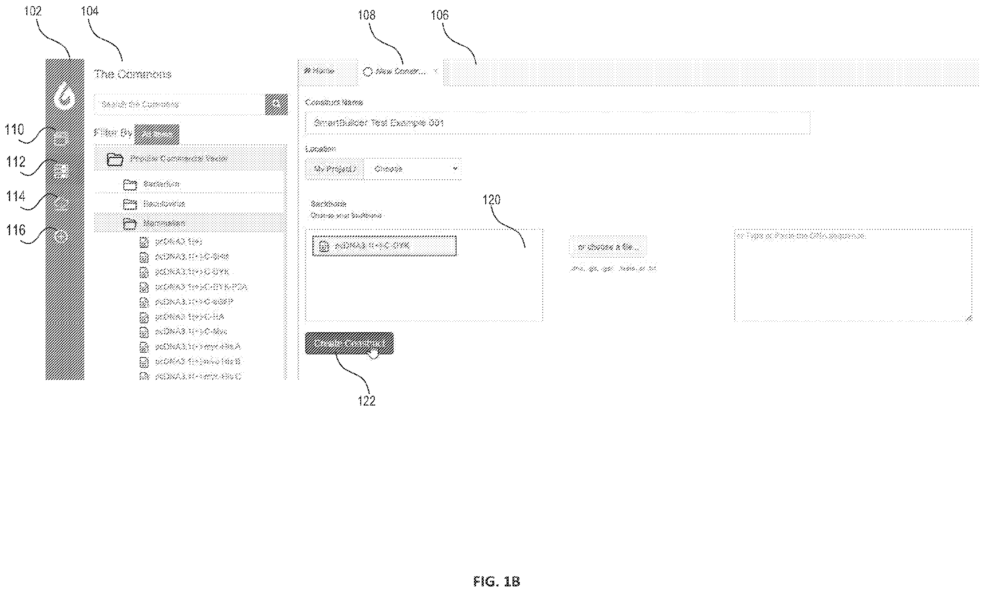

As depicted in FIG. 1B, the user expands the nested list in the database navigation section 104 to show a list of Mammalian vectors. Further, the user provides an input selecting a vector backbone, specifically, by providing a drag-and-drop input that drags a vector "pcDNA3.1(+)-C-DYK" from the database navigation section 104 into the "Backbone" command box 120 in the tab user interface 108 and selecting the "Create Construct" button 122.

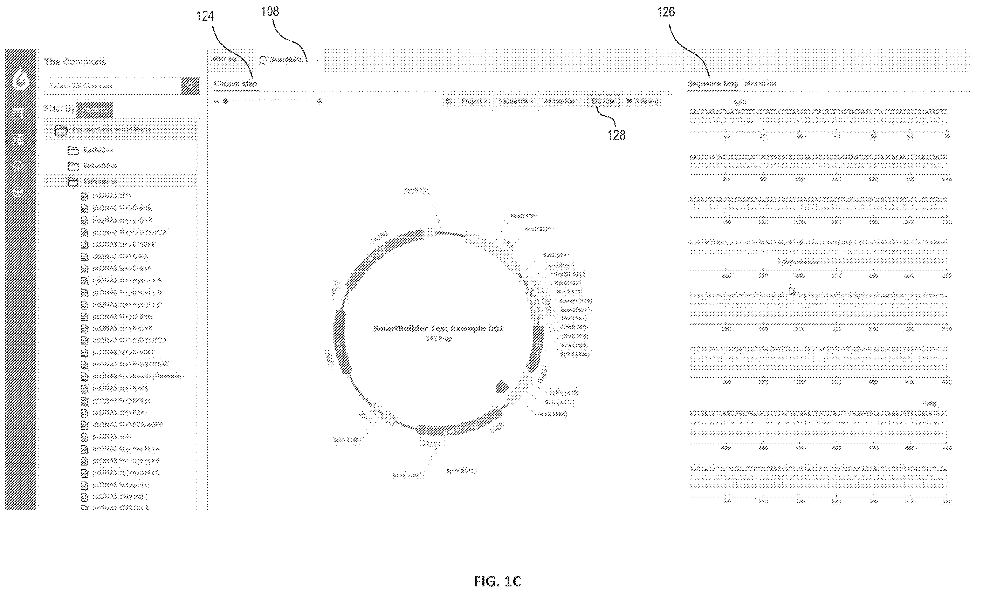

Turning to FIG. 1C, in response to the selection of the "Create Construct" button 122, the tab user interface 108 displays two graphical representations of the selected vector backbone: a circular map (or plasmid map) 124 and a sequence map 126. In the depicted example, the circular map is a visualization of the construct with functional parts annotated and displayed in color blocks while the rest regions are demonstrated as solid line. The color blocks on circular map represent validated parts, and can be easily selected by a click to trigger flash outline effect. The sequence map demonstrates the double strand DNA in base pair with the corresponding color blocks shown underneath.

As depicted in FIG. 1C, different types of parts can be distinguished with different visual characteristics (e.g., color, shape). For example, a flat petango in reddish orange (#f17c67) represents a coding sequence (AmpR), with orientation indicated by acute angle. In some examples, when displaying a graphical representation of a vector bone, the system automatically scans the vector bone to identify parts (e.g., based on the databases described with respect to FIG. 3) and displays the identified parts based on the corresponding visual characteristics.

The tab user interface 108 also includes various tools for facilitating the design process. Function descriptions of exemplary tools are provided below.

| **Category** | **Tool** | **Function** |
|---|---|---|
| Project | Duplicate project | Generate a copy of project |
| | Export PDF | Export PDF file of project |
| | Export.gb | Export .gb file of project |
| Sequence | Edit | Edit selected sequence |
| | Insert | Insert sequence at a certain position |
| | Delete | Delete selected sequence |
| Annotation | Add | Annotate selected sequence |
| | Edit | Edit existing annotation |
| | Remove | Remove existing annotation while keeping sequence |
| Enzyme | / | Show or hide enzyme upon click |
| Ordering | / | Click to place order via GenSmart^{™} online ordering or GenScript quote/ordering system |

For example, upon a user selection of the "Enzyme" button 128, a number of cloning sites, along with the corresponding locations, (e.g., "HindIII (911)", "SacI (923)") are displayed in the circular map 124, as shown in FIG. 1C.

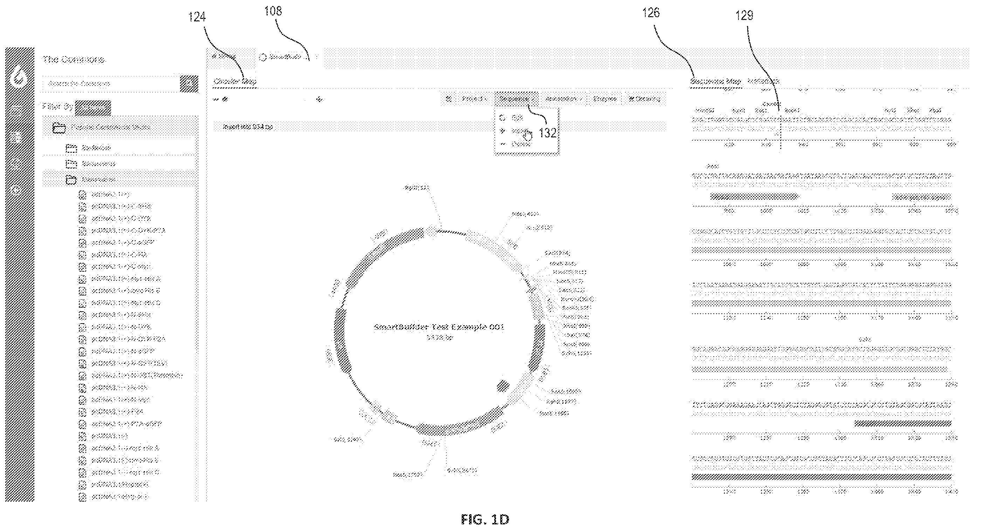

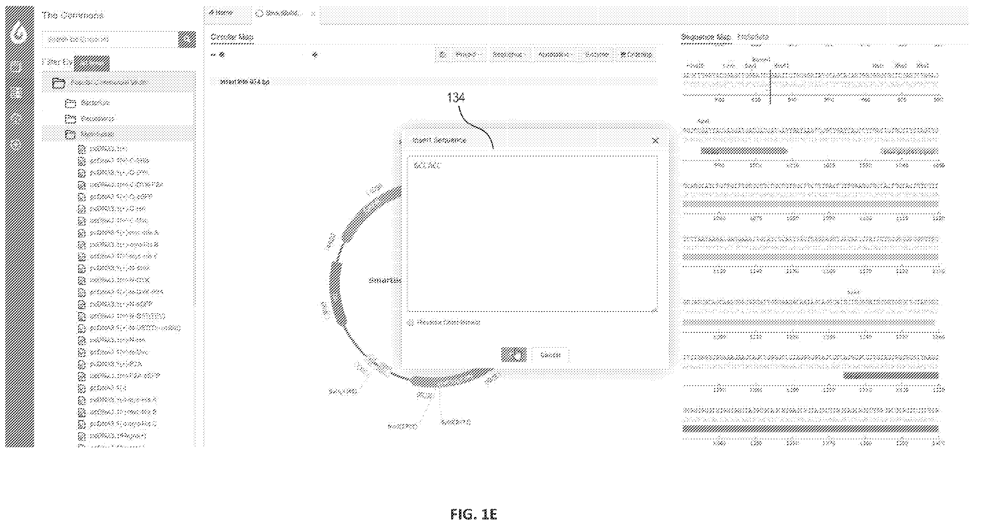

The system allows the user to easily modify the vector backbone in the tab user interface 108. Turning to FIG. 1D, the user selects a particular location on the vector backbone by selecting (e.g., clicking or tapping) a location on the sequence map 126, as indicated by cursor 129. The user then selects the "Sequence" button 132 to display a drop-down menu and selects the "Insert" option. As shown in FIG. 1E, a pop-up command box 134 is provided for the user to specify a sequence to insert at the selected location on the vector backbone. After the user selects the "OK" button on the command box 134, the sequence map and the circular map are automatically updated to show the inserted content.

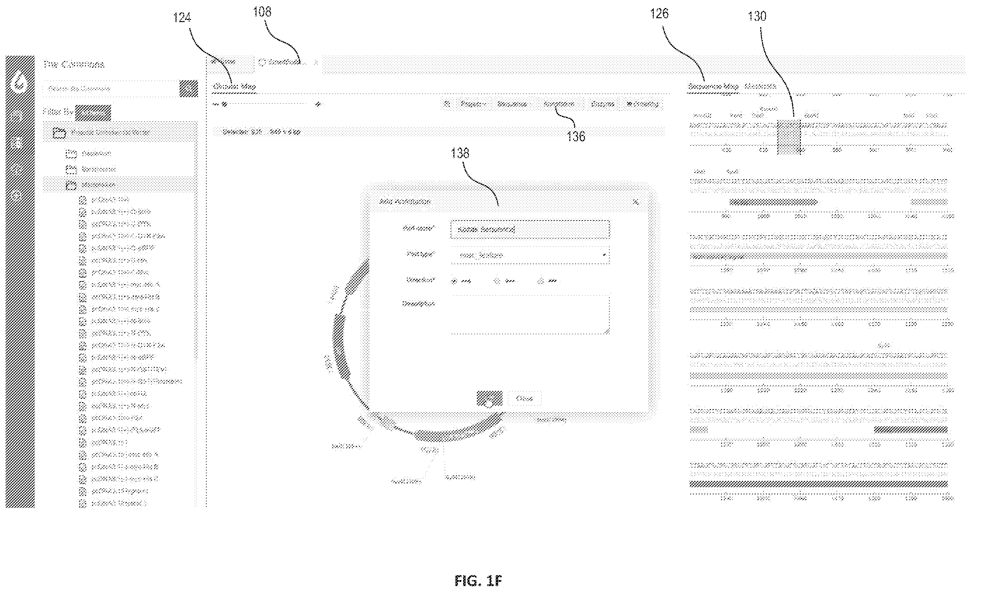

The system further allows the user to easily annotate any portion of the DNA construct. Turning to FIG. 1F, the user selects a portion of the vector backbone, as indicated by the highlighted portion 130 on the sequence map 126. The user then selects the "Annotation" button 136 to display a drop-down menu (not depicted) and selects an option for adding an annotation. In response, a pop-up command box 138 is provided for the user to annotate the selected portion of the vector backbone and specify properties to be associated with the annotated portion. After the user selects the "OK" button on the command box 138, the sequence map and the circular maps are automatically updated to show the selected portion as an annotated part (e.g., with a particular color or shape) based on the user-specified properties. In some examples, one or more user-specific databases are updated to include the annotated portion as a part.

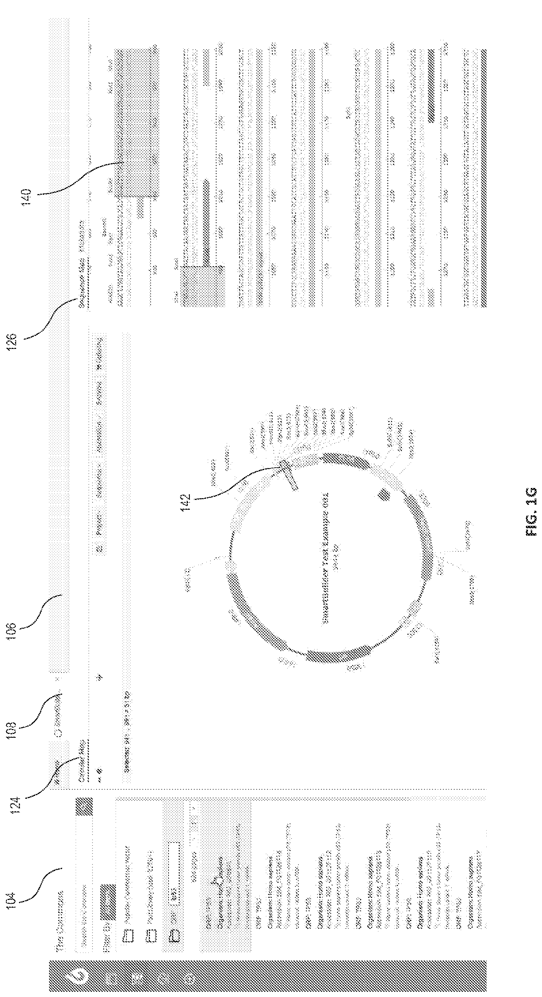

The system further allows the user to add a part to a vector backbone using natural and intuitive input techniques, such as a drag-and-drop input. With reference to FIG. 1G, in the database navigation section 104, the user searches for a gene of interest using the search term "tp53" in the ORF database to obtain a list of search results. Further, in the tab user interface 108, the user selects a portion of the vector backbone, as indicated by the highlighted portion 142 on the circular map 124 and the highlighted portion 140 on the sequence map 126. In the depicted example, the selected portion includes a group of adjacent cloning sites on the vector backbone. In some examples, the user makes the selection by interacting with either the circular map 124 (e.g., clicking or tapping a first region and dragging the cursor to a second region) or the sequence map 126 (e.g., double-clicking or double-tapping a region and dragging the cursor to a second region). The display of the two maps is synchronized such that a selection of one or more functional parts on one map is automatically reflected on the other map.

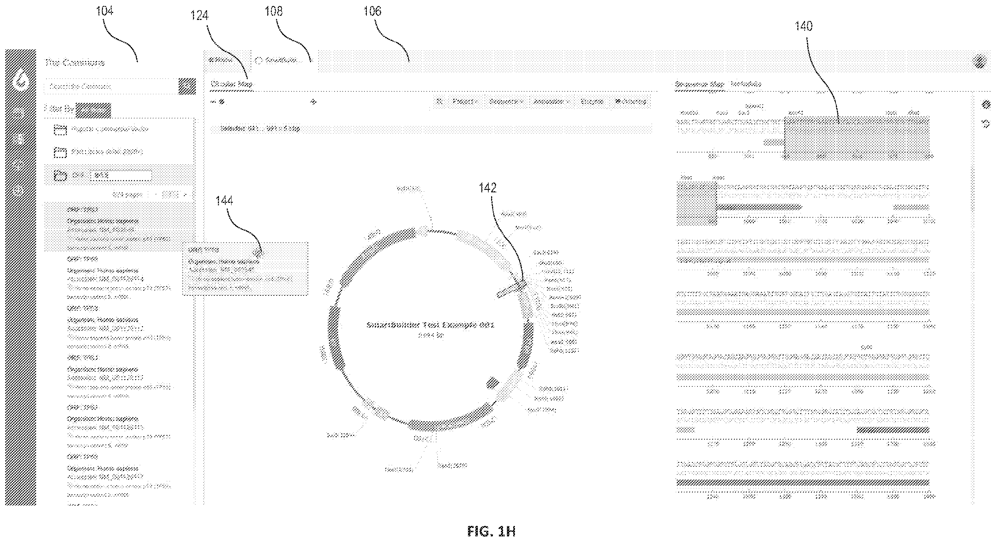

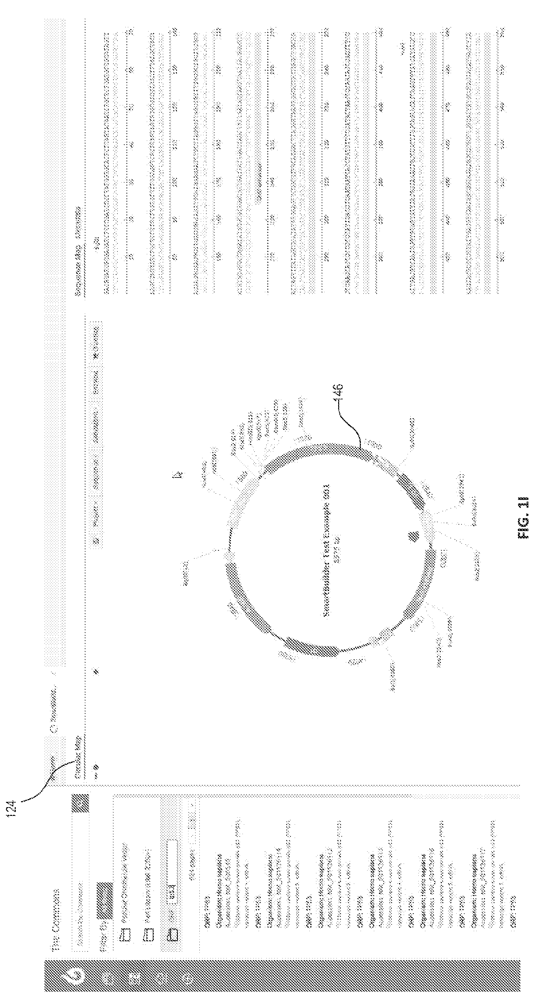

As depicted in FIG. 1H, the user drags the part "TP53" from the database navigation section 104 toward the tab user interface 108, as indicated by the cursor 144. When the user drops the part "TP53" onto the circular map 124, the system updates the vector backbone by inserting the part "TP53" into the vector backbone at one of the selected cloning sites. The system also automatically updates the circular map and the sequence map to display the updated vector backbone. As shown in FIG. 1I, the circular map 124 now shows a vector backbone having a part "TP53" 146.

In some examples, the drag-and-drop input comprises a click and drag input made with a mouse. For example, to drag an item, the user clicks on the item by depressing one or more buttons on the mouse and moves the mouse curser while holding the one or more buttons. To drop the item, the user moves the mouse curser to the desired location and releases the one or more buttons. In some examples, the drag-and-drop input comprises a tap and drag input made with a finger on a touch-sensitive display. For example, to drag an item, the user touches a displayed item using a finger to select the item and moves the finger on the touch-sensitive display. To drop the item, the user moves the finger to the desired location and lifts the finger away from the touch-sensitive display. In some examples, the system automatically scans the inserted part to identify properties associated with the part (e.g., type, direction, name) based on, for example, one or more of the databases described with respect to FIG. 3. The system then displays the part in accordance with visual characteristics (e.g., color) pre-associated with the identified part. It should be appreciated that the user can drop the dragged part on either the circular map or the sequence map to insert the dragged part to the vector backbone.

With reference to FIG. 1H, in some examples, the user does not need to drop the selected part specifically onto the selected portion 140 or 142 to achieve the insert operation. Rather, the user can simply drop the part on any area on the circular map or the sequence map, and the system can automatically determine, based on the part being dragged (i.e., a gene of interest) and the parts being selected on the vector backbone (i.e., multiple cloning sites), that the user intends to insert the dragged part into the vector backbone. Further, the system can identify one cloning site of the selected multiple cloning sites based on predefined criteria (e.g., the closest to where the dragged part is dropped) and insert the part at the identified cloning site. In some examples, the system can automatically make suggestions to the user regarding proper sites for inserting the dragged part.

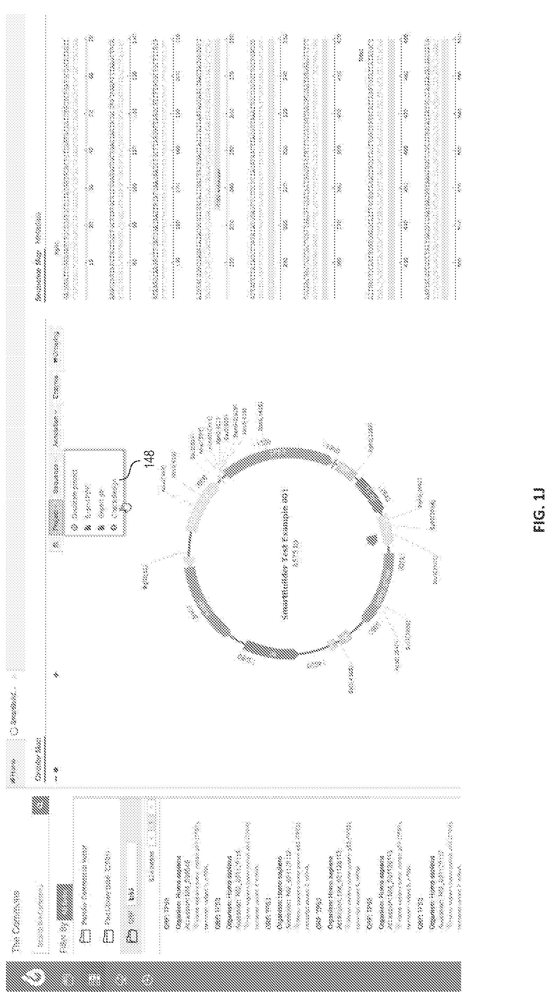

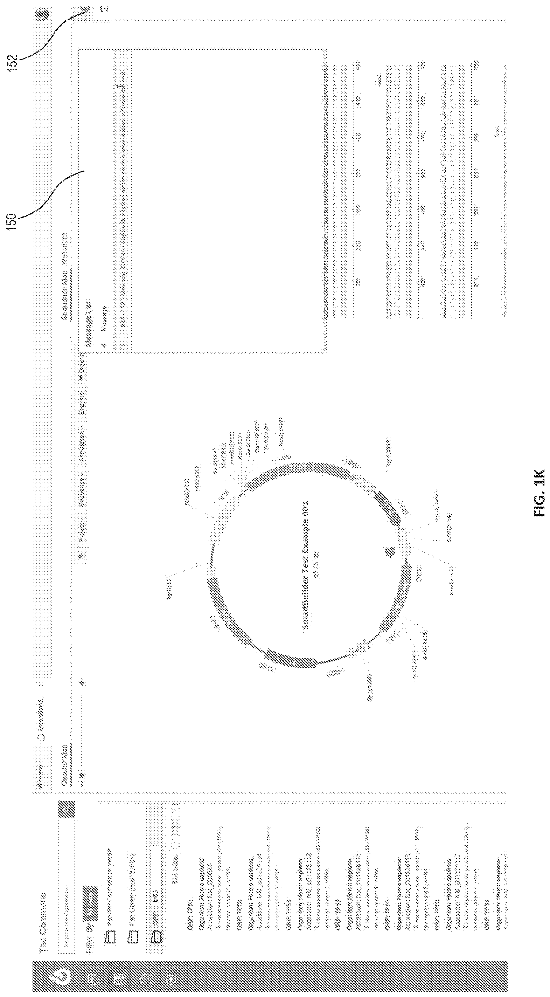

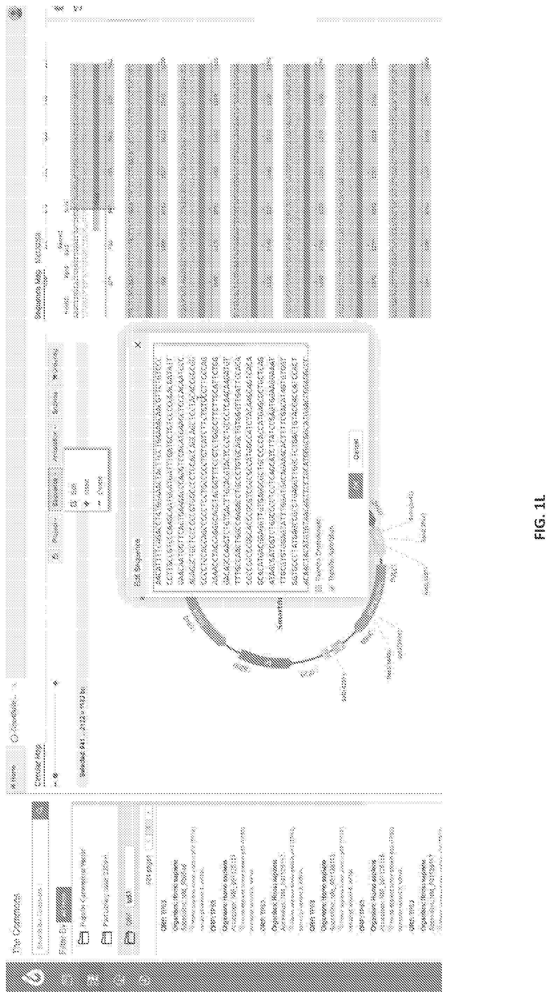

With reference to FIG. 1J, after modifying the vector backbone, the user initiates an error-checking request by selecting the "Check design" option 148. In response to receiving the request, the system executes one or more error-checking algorithms to identify an error with the updated vector backbone. Turning to FIG. 1K, when an error is identified, the system displays an icon 152 indicating that an error has been identified. Upon a user selection of the icon 152, a menu 150 that lists all identified errors is displayed. In some examples, interacting with (e.g., clicking, tapping) a displayed error message causes the system to display the sequence map or the circular map to display the portion of the DNA construct that contains the error. As such, the user can address the error by, for example, editing the portion, as shown in FIG. 1L.

In some examples, the system automatically executes error-checking algorithms without the user's selection of the "Check design" option. For example, the system can automatically execute error-checking algorithms when a certain type of change (e.g., insertion of a gene of interest) is made to the vector backbone. Further, when errors are identified, the system can automatically provide notifications of the identified errors and, if applicable, provide suggestions for correcting the errors.

FIGS. 2A-C illustrates exemplary techniques including UIs for replacing an existing part on a vector backbone with a different part. With reference to FIG. 2A, in the database navigation section 204, the user conducts a search using a search term "IL12" in the ORF database to obtain a list of search results. In the tab user interface 208, the user selects (e.g., by clicking or tapping) an existing part "TP53" on the vector backbone, as indicated by the outline 242 on the circular map 224 and the outline 240 on the sequence map 226. As discussed above, the user can make the selection on either the circular map or the sequence map (e.g., by clicking and double-clicking the color block, respectively) and the display of the two maps are synchronized such that making the selection on one map will be automatically reflected on the other map.

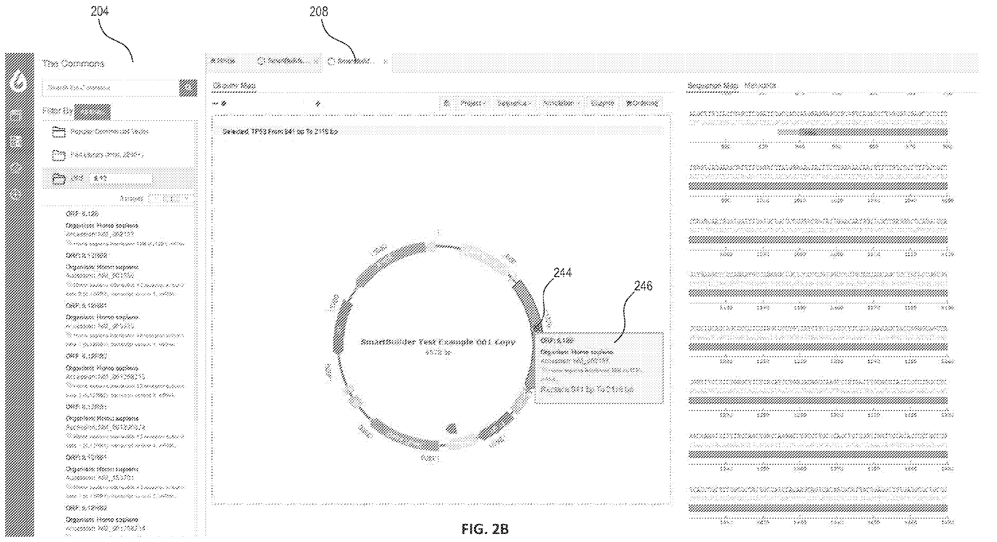

With reference to FIG. 2B, the user drags a part "IL12B", as indicated by cursor 244, from the database navigation section 204 toward the tab user interface 208. As depicted, while the user hovers the dragged part over the selected part "TP53" on the vector backbone, a pop-up message box 246 is displayed near the cursor, showing a message indicating the operation to be performed (i.e., "Replace 941 bp To 2119 bp").

When the user drops the dragged part "IL12B" onto the circular map, the system automatically updates the vector backbone by replacing the part "TP53" with the dragged part "IL12B". The system also automatically updates the circular map and the sequence map to display the updated vector backbone. As shown in FIG. 1C, the circular map 224 now shows a vector backbone having a part "IL128" at the location where "TP53" was displayed. It should be appreciated that the user can drop the dragged part on either the circular map or the sequence map to achieve the replace operation.

In some examples, the system automatically scans the dragged part "IL12B" to identify properties associated with the part (e.g., type, direction, name) based on, for example, one or more of the databases described with respect to FIG. 3. The system then displays the part in accordance with visual characteristics (e.g., color) pre-associated with the identified part.

With reference to FIG. 2B, in some examples, the user does not need to drop the dragged part "IL12B" directly onto the selected part "TP53" to achieve the replace operation. Rather, the user can simply drop the part on any area on the circular map or the sequence map, and the system can automatically determine, based on the part being dragged (i.e., a first gene of interest) and the part being selected on the vector backbone (i.e., a second gene of interest), that the user intends to replace the selected part on the vector backbone with the dragged part.

It should be appreciated that the UI flows described above are merely exemplary and that the system generally allows the user to provide inputs using natural and intuitive input techniques and is able to derive the intended operation based on such inputs. For example, the system can allow the user to copy an existing part on the vector backbone and insert the copied part at another location on the vector backbone. As another example, the user can drag a part from the database navigation section onto an existing part on the vector backbone even if the existing part is not pre-selected, and the system can derive the intended operation (e.g., insert, replace) based on the properties associated with the parts. It should be further appreciated that any of the user edits above (e.g., inserted parts, annotations) can be undone or cancelled.

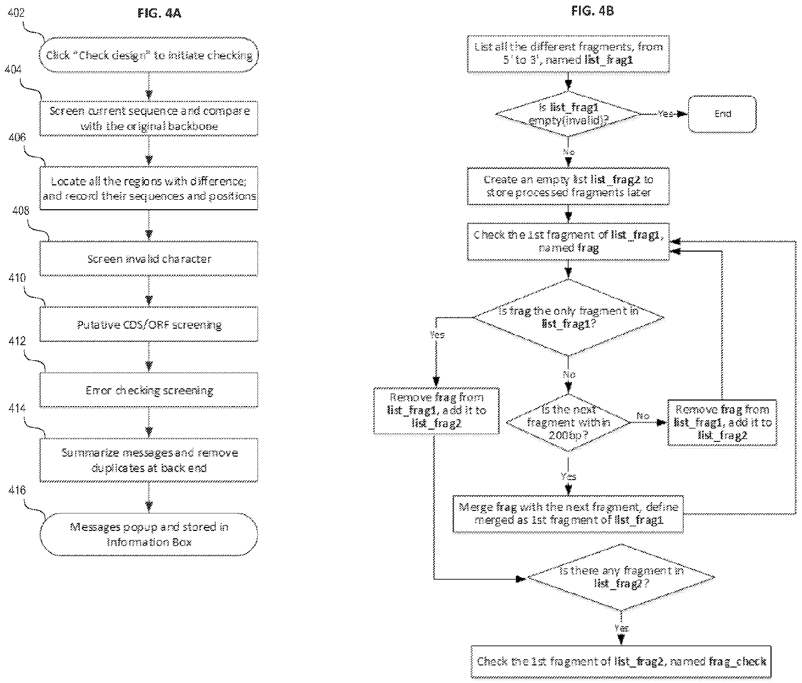

FIGS. 4A-D illustrate exemplary error-checking algorithms of the DNA construct design system, according to some examples. The ultimate goal of making a construct is to obtain a functional vehicle with biological purposes, which implies that the components (parts), their connections (orders), and the interactions are worth our knowledge, experience and time to check. Due to inconsistent levels of molecular background in the user base and the limitation of human power in checking base pairs represented by A/T/G/C, it is urgent to develop a computer program to relieve researchers from such tedious steps. Further, it is important to optimize the error-checking algorithms such that, when executed, the error-checking process is conducted in a comprehensive, accurate, and efficient manner. Thus, the error checking program is developed based on the summarization of rule sets.

FIG. 4A provides an overview of error-checking process. At block 402, the process starts when the user selects the "Check design" option, for example, as depicted in FIG. 1J. At block 404, the system screens the current sequence and compares the current sequence with the original vector backbone. At block 406, the system locates all the regions with differences and records the corresponding sequences and positions. At block 408, the system screens the current sequence for invalid characters (i.e., characters other than "A", "T", "G", "C" and "N"). At block 410, the system screens the current sequence for putative CDS/ORF. At block 412, the system screens the current sequence, including the identified putative CDS/ORF, for errors. At block 414, the system summarizes error messages and removes duplicates. At block 416, the process ends by displaying error messages.

FIG. 4B illustrates an exemplary process of defining sequences for checking as indicated in block 406 of FIG. 4A. As shown in FIG. 4B, the system records the original backbone and the current sequence to obtain the edited sequence which will be the target region(s) for further procedures. The interface screens for both 5' and 3' flanking regions in order to provide more accurate judgements on potential design errors. For example, if user inserts an ORF sequence into the position between BamHI and EcoRI of pcDNA 3.1(+)-C-DYK, the interface would check the 3' flanking region and then realize that Flag tag might be fused with the insertion, consequently, to check whether the stop codon of the ORF is deleted and the region between ORF and Flag tag is a multiplier of 3 to ensure in-frame fusion. In brief, the flanking regions of both terminuses will literally facilitate the error checking.

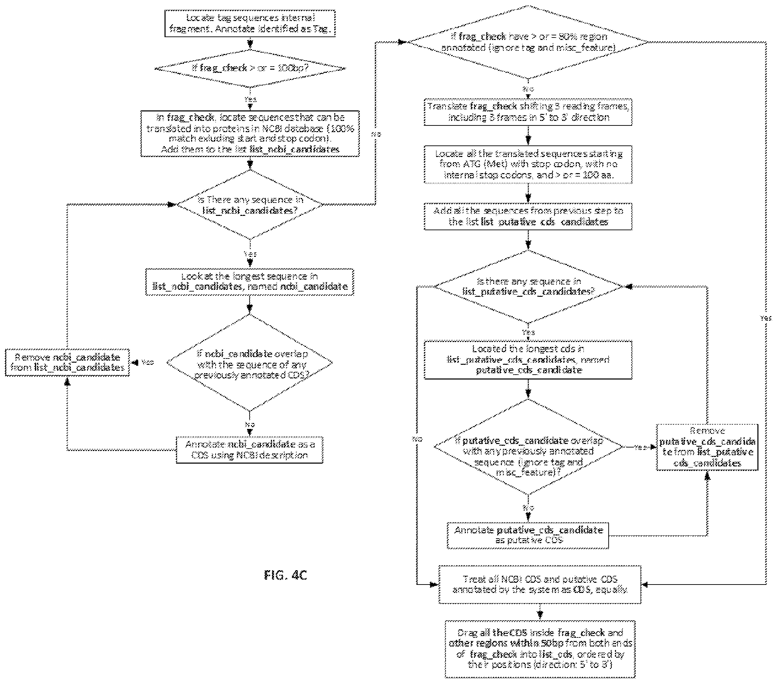

FIG. 4C illustrates an exemplary process of "putative CDS/ORF screening", which corresponds to block 410 of FIG. 4A. The system can annotate the target region(s) based on the databases described with respect to FIG. 3, for example, the human and mouse ORF database and the built-in part library consisting of thousands of parts. In addition, the system can screen for putative CDS by means of open reading frame frameshift translation (both 5' to 3' and 3' to 5') and recognize ORF by blast against NCBI ORFdatabase. ORF has been widely used for biological research, indicating that a tool integrated with ORF identifier function can improve researcher's efficiency by reducing the labor/time to annotate by blasting at NCBI website. In addition, ORF identifier and putative CDS screening are dual insurance to eliminate unintentional mistakes such as insertion and deletion.

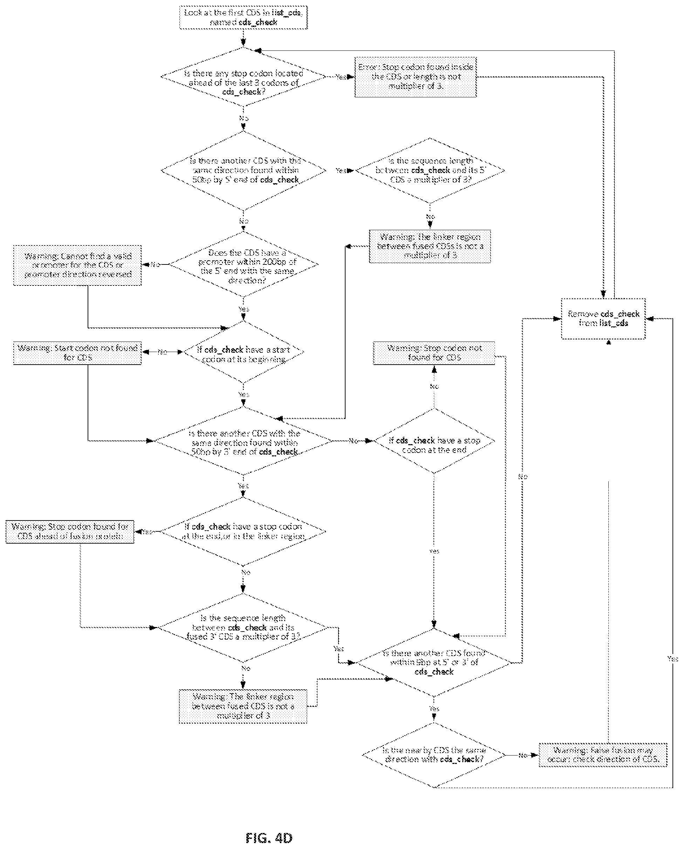

FIG. 4D illustrates an exemplary process of identifying potential functional errors that may affect the biological functions of the construct, which corresponds to block 412 of FIG. 4A. As depicted, the construct design logics and rules include checking whether there is any internal (e.g., stop codon) detected within a CDS or fused CDS, whether there is a valid promoter for the CDS and whether the direction of promoter and CDS is the same, whether there is a start codon, whether the fused protein of two or more CDS is in-frame (e.g., whether the distance between the two CDS is a multiplier of 3), and whether the fused CDSs are of the same direction.

There are three types of messages for indicating issues discovered in the error-checking process: Information, Warning, and Error. Error messages are issues that need to be corrected. Warning messages are issues that may affect biological functions of the construct. Information messages inform the user of invalid characters in the sequence which may lead to unsuccessful gene synthesis order placing. All error messages and warning messages are provided to the user, for example, via the menu 150 of FIG. 1K. Exemplary messages are provided below.

| **No.** | **Message** |
|---|---|
| 1 | Info.: Invalid nucleotide 'NNN' found in the sequence |
| 2 | Error: Stop codon found inside the CDS or length is not multiplier of 3 |
| 3 | Warning: The linker region between fused CDSs is not a multiplier of 3 |
| 4 | Warning: Stop codon not found for CDS |
| 5 | Warning: Stop codon found for CDS ahead of fusion protein |
| 6 | Warning: The linker region between fused CDS is not a multiplier of 3 |
| 7 | Warning: Cannot find a valid promoter for the CDS or promoter direction reversed |
| 8 | Warning: Start codon not found for CDS |
| 9 | Warning: False fusion may occur: check direction of CDS. |
| 10 | Error: No ATG found right after SD (Kozak sequence) |

FIGS. 5A-B illustrates processes 500 and 550, respectively, for providing a DNA construct design system, according to various examples. Processes 500 and/or 550 are performed, for example, using one or more electronic devices implementing a software platform. In some examples, processes 500 and/or 550 are performed using a client-server system, and the blocks of processes 500 and 550 are divided up in any manner between the server and a client device. In other examples, the blocks of processes 500 and/or 550 are divided up between the server and multiple client devices. In other examples, processes 500 and/or 550 are performed using only a client device (e.g., device 600) or only multiple client devices. In processes 500 and/or 550, some blocks are, optionally, combined, the order of some blocks is, optionally, changed, and some blocks are, optionally, omitted. In some examples, additional steps may be performed in combination with the processes 500 and/or 550. Accordingly, the operations as illustrated (and described in greater detail below) are exemplary by nature and, as such, should not be viewed as limiting.

In the process 500, at block 502, an electronic device with a display receives an input selecting a vector backbone, wherein the vector backbone comprises a plurality of functional parts. At block 504, in response to receiving the input selecting the vector backbone, the electronic device displays a graphical representation of the vector backbone. At block 506, the electronic device receives an input selecting one or more functional parts of the plurality of functional parts of the vector backbone. At block 508, after receiving the input selecting one or more functional parts on the vector backbone, the electronic device receives a drag-and-drop input comprising an indication of a functional part. At block 510, in response to receiving the drag-and-drop input, the electronic device updates the vector backbone based on the functional part indicated in the drag-and-drop input and the selected one or more functional parts of the plurality of functional parts of the vector backbone. At block 512, the electronic device displays a graphical representation of the updated vector backbone.

In the process 550, at block 552, an electronic device receives an error-checking request on a user-edited DNA construct, wherein the user-edited DNA construct is edited based on an original DNA construct. At block 554, in response to receiving the input, the electronic device identifies a set of sequences in the user-edited DNA construct, wherein the set of sequences is not present in the original DNA construct. At block 556, the electronic device identifies a presence of one or more coding sequences by comparing the set of sequences with a plurality of databases. At block 558, the electronic device identifies one or more errors in the identified one or more coding sequences based on a plurality of predefined rules. At block 560, the electronic device displays an output indicative of the one or more errors.

The operations described above with reference to FIGS. 1A-5B are optionally implemented by components depicted in FIG. 6. It would be clear to a person having ordinary skill in the art how other processes are implemented based on the components depicted in FIGS. 1A-5B.

FIG. 6 illustrates an example of a computing device in accordance with one embodiment. Device 600 can be a host computer connected to a network. Device 600 can be a client computer or a server. As shown in FIG. 6, device 600 can be any suitable type of microprocessor-based device, such as a personal computer, workstation, server or handheld computing device (portable electronic device) such as a phone or tablet. The device can include, for example, one or more of processor 610, input device 620, output device 630, storage 640, and communication device 660. Input device 620 and output device 630 can generally correspond to those described above, and can either be connectable or integrated with the computer.

Input device 620 can be any suitable device that provides input, such as a touch screen, keyboard or keypad, mouse, or voice-recognition device. Output device 630 can be any suitable device that provides output, such as a touch screen, haptics device, or speaker.

Storage 640 can be any suitable device that provides storage, such as an electrical, magnetic or optical memory including a RAM, cache, hard drive, or removable storage disk. Communication device 660 can include any suitable device capable of transmitting and receiving signals over a network, such as a network interface chip or device. The components of the computer can be connected in any suitable manner, such as via a physical bus or wirelessly.

Software 650, which can be stored in storage 640 and executed by processor 610, can include, for example, the programming that embodies the functionality of the present disclosure (e.g., as embodied in the devices as described above).

Software 650 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 640, that can contain or store programming for use by or in connection with an instruction execution system, apparatus, or device.

Software 650 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

Device 600 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Device 600 can implement any operating system suitable for operating on the network. Software 650 can be written in any suitable programming language, such as C, C++, Java or Python. In various embodiments, application software embodying the functionality of the present disclosure can be deployed in different configurations, such as in a client/server arrangement or through a Web browser as a Web-based application or Web service, for example.

## Claims

1. A computer-implemented method of designing a DNA construct, comprising:
at an electronic device with a display,
receiving an input selecting a vector backbone, wherein the vector backbone comprises a plurality of functional parts;
in response to receiving the input selecting the vector backbone, displaying a graphical representation of the selected vector backbone, wherein the graphical representation of the selected vector backbone includes a plasmid map and an optional sequence map;
receiving an input selecting one or more functional parts of the plurality of functional parts of the vector backbone;
after receiving the input selecting one or more functional parts on the vector backbone, receiving a drag-and-drop input comprising an indication of a functional part;
in response to receiving the drag-and-drop input, updating the vector backbone based on the functional part indicated in the drag-and-drop input and the selected one or more functional parts of the plurality of functional parts of the vector backbone, wherein (i) in case the one or more functional parts includes an existing gene on the vector backbone, updating the vector backbone comprises replacing the existing gene with the functional part indicated in the drag-and-drop input, and/or (ii) in case the one or more functional parts includes one or more cloning sites on the vector backbone, updating the vector backbone comprises inserting the functional part indicated in the drag-and-drop input at a cloning site of the one or more cloning sites on the vector backbone; and
displaying a graphical representation of the updated vector backbone.

2. The method of claim 1, wherein the drag-and-drop input comprises a click and drag input made with a mouse.

3. The method of claim 1, wherein the drag-and-drop input comprises a tap and drag input made with a finger on a touch-sensitive display.

4. The method of any of claims 1-3, further comprising:
receiving an input including a search term corresponding to a functional part;
identifying one or more search results based on a plurality of databases.

5. A computer-implemented method of error-checking a user-edited DNA construct, the method comprising:
receiving an error-checking request on the user-edited DNA construct;
in response to receiving the input, identifying a set of sequences in the user-edited DNA construct, wherein the set of sequences is not present in the original DNA construct;
identifying a presence of one or more coding sequences by comparing the set of sequences with a plurality of databases;
identifying one or more errors in the identified one or more coding sequences based on a plurality of predefined rules; and
displaying an output indicative of the one or more errors,
wherein the user-edited DNA construct is edited based on an original DNA construct according to the method of any one of claims 1-4.

6. The method of claim 5, wherein the output comprises: a textual output; a graphical output; an auditory output; or any combination thereof.

7. The method of claim 5 or 6, wherein identifying one or more errors comprises:
identifying one or more invalid characters in the set of sequences.

8. The method of any of claims 5-7, wherein the plurality of databases includes a user-specific database, a system-specific database, a public database, or any combination thereof.

9. The method of any of claims 5-8, wherein identifying one or more errors comprises:
determining whether a stop codon is present within a coding sequence of the one or more coding sequences; and /or
determining whether a start codon is present before a coding sequence of the one or more coding sequences; and /or
determining whether two coding sequences of the one or more coding sequences are within a predefined distance in the user-edited DNA construct and whether the two coding sequences are of a same direction.

10. The method of claim 9, further comprising:
in accordance with a determination that two coding sequences of the one or more coding sequences are within a predefined distance in the user-edited DNA construct and that the two coding sequences are of a same direction: determining the two coding sequences are in-frame; or
in accordance with a determination that two coding sequences of the one or more coding sequences are within a predefined distance in the user-edited DNA construct and that the two coding sequences are of a same direction: determining a stop codon is present between the two coding sequences.

11. The method of any of claims 5-10, wherein identifying one or more errors comprises:
determining whether a promoter is present within a predefined distance with a coding sequence of the one or more coding sequences; and
in accordance with a determination that a promoter is present within a predefined distance with a coding sequence of the one or more coding sequences: determining whether the promoter is of a same direction as the coding sequence.

12. An electronic device, comprising:
one or more processors;
a memory; and
one or more programs, wherein the one or more programs are stored in the memory and configured to be executed by the one or more processors, the one or more programs including instructions for:
receiving an error-checking request on a user-edited DNA construct;
in response to receiving the input, identifying a set of sequences in the user-edited DNA construct, wherein the set of sequences is not present in the original DNA construct;
identifying a presence of one or more coding sequences by comparing the set of sequences with a plurality of databases;
identifying one or more errors in the identified one or more coding sequences based on a plurality of predefined rules; and
displaying an output indicative of the one or more errors,
wherein the user-edited DNA construct is edited based on an original DNA construct according to the method of any one of claims 1-4.

## Patentansprüche

1. Computer-implementiertes Verfahren zum Konstruieren eines DNA-Konstrukts, umfassend:
an einer elektronischen Vorrichtung mit einer Anzeige,
Empfangen einer Eingabe, die ein Vektor-Backbone auswählt, wobei das Vektor-Backbone eine Vielzahl von funktionellen Komponenten umfasst;
in Reaktion auf das Empfangen der Eingabe, die das Vektor-Backbone auswählt, Anzeigen einer grafischen Darstellung des ausgewählten Vektor-Backbones, wobei die grafische Darstellung des ausgewählten Vektor-Backbones eine Plasmidkarte und eine optionale Sequenzkarte enthält;
Empfangen einer Eingabe, die eine oder mehrere funktionelle Komponenten aus der Vielzahl von funktionellen Komponenten des Vektor-Backbones auswählt;
nach dem Empfangen der Eingabe, die eine oder mehrere funktionelle Komponenten auf dem Vektor-Backbone auswählt, Empfangen einer Drag-and-Drop-Eingabe, die eine Angabe einer funktionellen Komponente umfasst;
in Reaktion auf das Empfangen der Drag-and-Drop-Eingabe, Aktualisieren des Vektor-Backbones basierend auf der in der Drag-and-Drop-Eingabe angegebenen funktionellen Komponente und den ausgewählten ein oder mehreren funktionellen Komponenten aus der Vielzahl von funktionellen Komponenten des Vektor-Backbones, wobei (i) für den Fall, dass die ein oder mehreren funktionellen Komponenten ein vorhandenes Gen auf dem Vektor-Backbone aufweisen, das Aktualisieren des Vektor-Backbones umfasst, das vorhandene Gen durch die in der Drag-and-Drop-Eingabe angegebene funktionelle Komponente zu ersetzen, und/oder (ii) für den Fall, dass die ein oder mehreren funktionellen Komponenten eine oder mehrere Klonierungsstellen auf dem Vektor-Backbone aufweisen, das Aktualisieren des Vektor-Backbones umfasst, die in der Drag-and-Drop-Eingabe angegebene funktionelle Komponente an einer Klonierungsstelle der ein oder mehreren Klonierungsstellen auf dem Vektor-Backbone einzusetzen; und
Anzeigen einer grafischen Darstellung des aktualisierten Vektor-Backbones.

2. Verfahren nach Anspruch 1, wobei die Drag-and-Drop-Eingabe eine Eingabe per Anklicken und Ziehen mit einer Maus umfasst.

3. Verfahren nach Anspruch 1, wobei die Drag-and-Drop-Eingabe eine Eingabe per Antippen und Ziehen mit einem Finger auf einer berührungsempfindlichen Anzeige umfasst.

4. Verfahren nach einem der Ansprüche 1-3, ferner umfassend:
Empfangen einer Eingabe, die einen Suchbegriff enthält, der einer funktionellen Komponente entspricht;
Identifizieren eines oder mehrerer Suchergebnisse basierend auf einer Vielzahl von Datenbanken.

5. Computer-implementiertes Verfahren zur Fehlerprüfung eines vom Benutzer bearbeiteten DNA-Konstrukts, wobei das Verfahren umfasst:
Empfangen einer Fehlerprüfungsanfrage für das vom Benutzer bearbeitete DNA-Konstrukt;
in Reaktion auf das Empfangen der Eingabe, Identifizieren einer Reihe von Sequenzen in dem vom Benutzer bearbeiteten DNA-Konstrukt, wobei die Reihe von Sequenzen in dem ursprünglichen DNA-Konstrukt nicht vorhanden ist;
Identifizieren eines Vorhandenseins einer oder mehrerer codierender Sequenzen durch Vergleichen der Reihe von Sequenzen mit einer Vielzahl von Datenbanken;
Identifizieren eines oder mehrerer Fehler in den identifizierten ein oder mehreren codierenden Sequenzen basierend auf einer Vielzahl von vordefinierten Regeln; und
Anzeigen einer Ausgabe, die auf die ein oder mehreren Fehler hinweist,
wobei das vom Benutzer bearbeitete DNA-Konstrukt basierend auf einem ursprünglichen DNA-Konstrukt gemäß dem Verfahren nach einem der Ansprüche 1-4 bearbeitet wird.

6. Verfahren nach Anspruch 5, wobei die Ausgabe umfasst: eine Textausgabe; eine grafische Ausgabe; eine akustische Ausgabe; oder eine beliebige Kombination davon.

7. Verfahren nach Anspruch 5 oder 6, wobei das Identifizieren eines oder mehrerer Fehler umfasst: Identifizieren eines oder mehrerer ungültiger Zeichen in der Reihe von Sequenzen.

8. Verfahren nach einem der Ansprüche 5-7, wobei die Vielzahl von Datenbanken eine benutzerspezifische Datenbank, eine systemspezifische Datenbank, eine öffentliche Datenbank oder eine beliebige Kombination davon umfasst.

9. Verfahren nach einem der Ansprüche 5-8, wobei das Identifizieren eines oder mehrerer Fehler umfasst:
Bestimmen, ob ein Stoppcodon innerhalb einer codierenden Sequenz der ein oder mehreren codierenden Sequenzen vorhanden ist; und/oder
Bestimmen, ob ein Startcodon vor einer codierenden Sequenz der ein oder mehreren codierenden Sequenzen vorhanden ist; und/oder
Bestimmen, ob zwei codierende Sequenzen der ein oder mehreren codierenden Sequenzen innerhalb eines vordefinierten Abstands in dem vom Benutzer bearbeiteten DNA-Konstrukt liegen und ob die beiden codierenden Sequenzen dieselbe Richtung haben.

10. Verfahren nach Anspruch 9, ferner umfassend:
entsprechend einer Bestimmung, dass zwei codierende Sequenzen der ein oder mehreren codierenden Sequenzen innerhalb eines vordefinierten Abstands in dem vom Benutzer bearbeiteten DNA-Konstrukt liegen und dass die beiden codierenden Sequenzen dieselbe Richtung haben:
Bestimmen, dass die beiden codierenden Sequenzen in demselben Leserahmen sind; oder
entsprechend einer Bestimmung, dass zwei codierende Sequenzen der ein oder mehreren codierenden Sequenzen innerhalb eines vordefinierten Abstands in dem vom Benutzer bearbeiteten DNA-Konstrukt liegen und dass die beiden codierenden Sequenzen dieselbe Richtung haben:
Bestimmen, dass zwischen den beiden codierenden Sequenzen ein Stoppcodon vorhanden ist.

11. Verfahren nach einem der Ansprüche 5-10, wobei das Identifizieren eines oder mehrerer Fehler umfasst:
Bestimmen, ob ein Promotor innerhalb eines vordefinierten Abstands zu einer codierenden Sequenz der ein oder mehreren codierenden Sequenzen vorhanden ist; und
entsprechend einer Bestimmung, dass ein Promotor innerhalb eines vordefinierten Abstands zu einer codierenden Sequenz der ein oder mehreren codierenden Sequenzen vorhanden ist: Bestimmen, ob der Promotor dieselbe Richtung hat wie die codierende Sequenz.

12. Elektronische Vorrichtung, umfassend:
einen oder mehrere Prozessoren;
einen Speicher; und
ein oder mehrere Programme, wobei die ein oder mehreren Programme im Speicher gespeichert und dafür ausgelegt sind, durch die ein oder mehreren Prozessoren ausgeführt zu werden, wobei die ein oder mehreren Programme Anweisungen enthalten zum:
Empfangen einer Fehlerprüfungsanfrage für ein vom Benutzer bearbeitetes DNA-Konstrukt;
in Reaktion auf das Empfangen der Eingabe, Identifizieren einer Reihe von Sequenzen in dem vom Benutzer bearbeiteten DNA-Konstrukt, wobei die Reihe von Sequenzen in dem ursprünglichen DNA-Konstrukt nicht vorhanden ist;
Identifizieren eines Vorhandenseins einer oder mehrerer codierender Sequenzen durch Vergleichen der Reihe von Sequenzen mit einer Vielzahl von Datenbanken;
Identifizieren eines oder mehrerer Fehler in den identifizierten ein oder mehreren codierenden Sequenzen basierend auf einer Vielzahl von vordefinierten Regeln; und
Anzeigen einer Ausgabe, die auf die ein oder mehreren Fehler hinweist,
wobei das vom Benutzer bearbeitete DNA-Konstrukt basierend auf einem ursprünglichen DNA-Konstrukt gemäß dem Verfahren nach einem der Ansprüche 1-4 bearbeitet wird.

## Revendications

1. Procédé mis en œuvre par ordinateur pour concevoir une construction d'ADN, le procédé comprenant les étapes suivantes :
au niveau d'un dispositif électronique doté d'un écran,
recevoir une entrée sélectionnant un squelette de vecteur, où le squelette de vecteur comprend une pluralité de parties fonctionnelles ;
en réponse à la réception de l'entrée sélectionnant le squelette de vecteur, afficher une représentation graphique du squelette de vecteur sélectionné, où la représentation graphique du squelette de vecteur sélectionné comprend une carte de plasmide et une carte de séquence optionnelle ;
recevoir une entrée sélectionnant une ou plusieurs parties fonctionnelles parmi la pluralité de parties fonctionnelles du squelette de vecteur ;
après la réception de l'entrée sélectionnant une ou plusieurs parties fonctionnelles sur le squelette de vecteur, recevoir une entrée par glisser-déposer comprenant une indication d'une partie fonctionnelle ;
en réponse à la réception de l'entrée par glisser-déposer, mettre à jour le squelette de vecteur sur la base de la partie fonctionnelle indiquée dans l'entrée par glisser-déposer et des une ou plusieurs parties fonctionnelles sélectionnées parmi la pluralité de parties fonctionnelles du squelette de vecteur, où (i) dans le cas où les une ou plusieurs parties fonctionnelles comprennent un gène existant sur le squelette de vecteur, la mise à jour du squelette de vecteur comprend le remplacement du gène existant par la partie fonctionnelle indiquée dans l'entrée par glisser-déposer, et/ou (ii) dans le cas où les une ou plusieurs parties fonctionnelles comprennent un ou plusieurs sites de clonage sur le squelette de vecteur, la mise à jour du squelette de vecteur comprend l'insertion de la partie fonctionnelle indiquée dans l'entrée par glisser-déposer au niveau d'un site de clonage parmi les un ou plusieurs sites de clonage sur le squelette de vecteur ; et
afficher une représentation graphique du squelette de vecteur mis à jour.

2. Procédé selon la revendication 1, dans lequel l'entrée par glisser-déposer comprend une entrée par clic et glisser effectuée à l'aide d'une souris.

3. Procédé selon la revendication 1, dans lequel l'entrée par glisser-déposer comprend une entrée par tapotement et glisser effectuée avec un doigt sur un écran tactile.

4. Procédé selon l'une quelconque des revendications 1 à 3, comprenant en outre les étapes suivantes :
recevoir une entrée comprenant un terme de recherche correspondant à une partie fonctionnelle ;
identifier un ou plusieurs résultats de recherche sur la base d'une pluralité de bases de données.

5. Procédé mis en œuvre par ordinateur pour vérifier les erreurs dans une construction d'ADN éditée par un utilisateur, le procédé comprenant les étapes suivantes :
recevoir une demande de vérification d'erreurs de la construction d'ADN éditée par l'utilisateur ;
en réponse à la réception de l'entrée, identifier un ensemble de séquences dans la construction d'ADN éditée par l'utilisateur, où l'ensemble de séquences n'est pas présent dans la construction d'ADN d'origine ;
identifier la présence d'une ou plusieurs séquences codantes en comparant l'ensemble de séquences avec une pluralité de bases de données ;
identifier une ou plusieurs erreurs dans les une ou plusieurs séquences codantes identifiées sur la base d'une pluralité de règles prédéfinies ; et
afficher une sortie indicative des une ou plusieurs erreurs,
dans lequel la construction d'ADN éditée par l'utilisateur est éditée sur la base d'une construction d'ADN d'origine selon le procédé de l'une quelconque des revendications 1 à 4.

6. Procédé selon la revendication 5, dans lequel la sortie comprend : une sortie textuelle ; une sortie graphique ; une sortie auditive ; ou toute combinaison de celles-ci.

7. Procédé selon la revendication 5 ou la revendication 6, dans lequel l'identification d'une ou plusieurs erreurs comprend :
l'identification d'un ou plusieurs caractères non valides dans l'ensemble de séquences.

8. Procédé selon l'une quelconque des revendications 5 à 7, dans lequel la pluralité de bases de données comprend une base de données spécifique à l'utilisateur, une base de données spécifique au système, une base de données publique ou toute combinaison de celles-ci.

9. Procédé selon l'une quelconque des revendications 5 à 8, dans lequel l'identification d'une ou plusieurs erreurs comprend les erreurs suivantes :
déterminer la présence éventuelle d'un codon d'arrêt dans une séquence codante parmi les une ou plusieurs séquences codantes ; et/ou
déterminer la présence éventuelle d'un codon de départ avant une séquence codante parmi les une ou plusieurs séquences codantes ; et/ou
déterminer la présence éventuelle de deux séquences codantes parmi les une ou plusieurs séquences codantes à une distance prédéfinie dans la construction d'ADN éditée par l'utilisateur et si les deux séquences codantes sont, ou non, dans la même direction.

10. Procédé selon la revendication 9, comprenant en outre les étapes suivantes :
conformément à la détermination selon laquelle deux séquences codantes parmi les une ou plusieurs séquences codantes se trouvent à une distance prédéfinie dans la construction d'ADN éditée par l'utilisateur et selon laquelle les deux séquences codantes sont dans la même direction : déterminer si les deux séquences codantes sont en phase de lecture (in-frame) ; ou
conformément à la détermination selon laquelle deux séquences codantes parmi les une ou plusieurs séquences codantes se trouvent à une distance prédéfinie dans la construction d'ADN éditée par l'utilisateur et selon laquelle les deux séquences codantes sont dans la même direction : déterminer si un codon d'arrêt est présent entre les deux séquences codantes.

11. Procédé selon l'une quelconque des revendications 5 à 10, dans lequel l'identification d'une ou plusieurs erreurs comprend les étapes suivantes :
déterminer la présence éventuelle d'un promoteur à une distance prédéfinie d'une séquence codante parmi les une ou plusieurs séquences codantes ; et
conformément à une détermination selon laquelle un promoteur est présent à une distance prédéfinie d'une séquence codante parmi les une ou plusieurs séquences codantes : déterminer si le promoteur à la même direction que la séquence codante.

12. Dispositif électronique, comprenant :
un ou plusieurs processeurs ;
une mémoire ; et
un ou plusieurs programmes, les un ou plusieurs programmes étant stockés dans la mémoire et configurés pour être exécutés par les un ou plusieurs processeurs,
les un ou plusieurs programmes comprenant des instructions pour :
recevoir une demande de vérification d'erreurs sur une construction d'ADN éditée par l'utilisateur ;
en réponse à la réception de l'entrée, identifier un ensemble de séquences dans la construction d'ADN éditée par l'utilisateur, où l'ensemble de séquences n'est pas présent dans la construction d'ADN d'origine ;
identifier la présence d'une ou plusieurs séquences codantes en comparant l'ensemble de séquences à une pluralité de bases de données ;
identifier une ou plusieurs erreurs dans les une ou plusieurs séquences codantes identifiées sur la base d'une pluralité de règles prédéfinies ; et
afficher une sortie indicative des une ou plusieurs erreurs,
dans lequel la construction d'ADN éditée par l'utilisateur est éditée sur la base d'une construction d'ADN d'origine selon le procédé de l'une quelconque des revendications 1 à 4.
